# EUROPÄISCHE PATENTANMELDUNG

(11) **EP 0 779 101 A1**
(43) Veröffentlichungstag der Anmeldung: **18.06.1997**
(21) Anmeldenummer: 96119692.0
(22) Anmeldetag: 09.12.1996
(51) Int. Cl.: B01J 31/04, B01J 31/02, C07C 41/03

(54) **Precursor für Alkoxylierungskatalysatoren**

(30) Priorität: 15.12.1995 DE 19546946
(71) Anmelder: HOECHST AKTIENGESELLSCHAFT, 65929 Frankfurt am Main (DE)
(72) Erfinder: Mayer, Michael. Dr., 63773 Goldbach (DE); Hess, Joachim, 65719 Hofheim (DE); Babiel, Alfred, 65606 Villmar (DE)

(57) **Zusammenfassung**

Precursor für Alkoxylierungskatalysatoren, hergestellt durch Umsetzung eines chelatisierenden anionischen oder nicht-ionischen Phasentransferkatalysators mit einer Erdalkaliverbindung in Gegenwart einer Verbindung, die oxalkyliert werden kann. Als Phasentransferkatalysator kommen dabei insbesondere oxethylierte Fettalkohole, Ethercarbonsäuren, Ethersulfonsäure oder Etherphosphonsäuren in Frage. Durch Zugabe von Schwefelsäure wird aus einem solchen Precursor ein Erdalkalisulfato-Komplex freigesetzt, der den eigentlichen Katalysator für die Alkoxylierung darstellt.

## Beschreibung

Bei der herkömmlichen Oxalkylierung, beispielsweise von Fettalkoholen und Aminen, entstehen Oxalkylate mit einer breiten Molekulargewichtsverteilung. Eine engere Molekulargewichtsverteilung läßt sich erreichen, wenn man sogenannte narrow-range Katalysatoren nimmt. Man erreicht damit eine Verringerung der Anteile am nicht oxalkylierten Ausgangsprodukt und eine Verringerung des Anteils an hochoxalkylierten Produkten. Als narrow-range Katalysatoren sind bereits Erdalkalisalze von organischen Carbonsäuren (US 4 282 387; EP 85 107), Erdalkalisalze von Ethercarbonsäuren (EP 295 578) und Erdalkalisalze von vicinal hydroxy, alkoxy-substituierten Fettsäuren (DE 3 706 047) beschrieben. All diesen Katalysatoren ist gemeinsam, daß es sich um definierte Erdalkalisalze handelt, die separat hergestellt werden und in dieser Form zu der Oxalkylierungsreaktion zugegeben werden. Daneben ist es auch bekannt, Calciumsulfat als narrow-range Katalysator zu verwenden (EP-A-0 301 621).

Es wurde nun gefunden, daß sich als narrow-range Katalysator auch ein Erdalkali-sulfato-Komplex eignet, der unmittelbar vor der Oxalkylierungsreaktion aus einem sogenannten Precursor, der eine Erdalkaliverbindung enthält, durch Zugabe von Schwefelsäure erzeugt wird.

Gegenstand der Erfindung ist ein Precursor für Alkoxylierungskatalysatoren, wobei dieser Precursor hergestellt wird durch Umsetzung eines chelatisierenden anionischen oder nicht-ionischen Phasentransferkatalysators mit einer Endalkaliverbindung in Gegenwart einer Verbindung, die oxalkyliert werden kann. Als chelatisierender anionischer oder nicht-ionischer Phasentransferkatalysator kommen vor allem Verbindungen der Formel 1

C₈-C₂₂-Alkyl-O(AO)ₓ-B (1)

in Frage, worin
- A: -C₂H₄- oder -C₃H₇-,
- B: Wasserstoff oder eine Gruppe der Formeln -CH₂COOH, -SO₃H oder - PO(OH)₂ und
- X: eine Zahl von 2 bis 10 bedeutet.

Als Verbindung, die oxalkyliert werden kann, nimmt man hier sinnvollerweise die gleiche Verbindung, die mit Hilfe dieses Precursors bzw. des daraus hergestellten Katalysators anschließend oxalkyliert werden soll. Als derartige Verbindungen kommen im Prinzip alle Verbindungen in Frage, die aktiven Wasserstoff enthalten, wie beispielsweise Monoalkohole, Polyalkohole (Glykole) oder Alkylphenole. Bavorzugt sind gesättigte oder ungesättigte Fettalkohole, vorzugsweise mit C₈-C₁₈-Atomen oder deren Mischungen wie beispielsweise Talgfettalkohol, Laurylalkohol, Stearylalkohol oder Oxoalkohole. Als Erdalkaliverbindung sind Calciumverbindungen bevorzugt, wie etwa Calciumoxid, Calciumhydroxid, Calciumcarbonat, Calciumchlorid, Calciumacetat oder andere Calciumsalze. Bei den Verbindungen der Formel (1) handelt es sich um oxalkylierte Fettalkohole (B = H) oder um die daraus abgeleiteten Ethercarbonsäuren, Ethersulfonsäuren oder Etherphosphonsäuren.

Die einzelnen Komponenten für den Precursor werden im allgemeinen in folgenden molaren Mengenverhältnissen eingesetzt: 0,1 bis 3, vorzugsweise 0,1 bis 0,5 Mol der Verbindung, die oxalkyliert werden kann, zusammen mit 0,4 bis 2,5, vorzugsweise 0,4 bis 1 Mol einer Verbindung der Formel (1) mit der Bedeutung B = Wasserstoff oder 2 bis 2,5, vorzugsweise 2,2 Mol der Verbindung, die oxalkyliert werden kann, zusammen mit 1,5 bis 1,95, vorzugsweise 1,7 bis 1,8 Mol einer Ethercarbonsäure, Ethersulfonsäure oder Etherphosphonsäure als Verbindung der Formel (1). Diese molaren Mengenangaben beziehen sich jeweils auf 1 Mol des Erdalkali-Kations.

Bei der Herstellung des Precursors erfolgt eine Umsetzung der Erdalkaliverbindung mit der Verbindung der Formel (1). Dies geschieht, indem man eine Mischung dieser beiden Komponenten bei Raumtemperatur zusammen mit der Verbindung, die oxalkyliert werden kann, verrührt, wobei sich, wenn man eine Verbindung der Formel (1) mit B = Wasserstoff nimmt, das entsprechende Alkoholat bildet. In den anderen Fällen bildet sich das Erdalkalisalz der Ethercarbonsäure, Ethersulfonsäure oder Etherphosphonsäure. Für die Bildung dieser Salze genügen bereits relativ kurze Reaktionszeiten in der Größenordnung von 30 Minuten. Für die Bildung der Alkoholate hat es sich dagegen als zweckmäßig erwiesen, die Umsetzung über einen längeren Zeitraum (bis zu 5 Stunden) und bei höheren Temperaturen (ca. 100 bis 120°C) durchzuführen. Wasserlösliche Calciumsalze wie beispielsweise Calciumacetat oder Calciumchlorid werden in Form einer wäßrigen Lösung eingesetzt, unlösliche oder schwerlösliche Calciumverbindungen wie Calciumcarbonat oder Calciumhydroxid werden als Feststoffe zugegeben. Bei Calciumoxid ist es von Vorteil, eine kleine Menge Wasser zuzugeben, um das Calciumoxid zumindest partiell in Calciumhydroxid umzuwandeln. Nach Beendigung der Reaktion wird das vorhandene Wasser destillativ abgetrennt, vorzugsweise mit Hilfe eines Schleppmittels wie Xylol oder Toluol. Nach Entfernung des Wassers wird die Restmenge dieses Schleppmittels ebenfalls destillativ entfernt. Im allgemeinen geht man so vor, daß man das Schleppmittel, beispielsweise Xylol, dem Reaktionsgemisch schon vor Beginn der Reaktion zugibt. Falls bei der Herstellung des Precursors nur geringe Mengen an Wasser anfallen, kann diese Restmenge im Precursor verbleiben. Es wird dann nach der Herstellung des eigentlichen Katalysators und vor der Oxalkylierungsreaktion entfernt. Zur Entfernung eventuell noch vorhandener Restmengen an Feststoffen empfiehlt es sich, den fertigen Precursor zu filtrieren.

Bei dem so hergestellten Precursor handelt es sich um eine homogene Lösung, die sehr gut lagerstabil ist. Aus diesem Precursor wird unmittelbar vor der Oxalkylierung der eigentliche Oxalkylierungskatalysator in Form eines Erdalkalisulfato-Komplexes durch Zugabe von Schwefelsäure freigesetzt. Erst diese Zugabe von Schwefelsäure erzeugt den funktionstüchtigen Katalysator. Die Menge an Schwefelsäure ist so zu bemessen, daß das molare Verhältnis von Sulfationen zu Erdalkalikationen einen Wert von 0,8 : 1 nicht überschreitet. Zur Vervollständigung dieser Reaktion rührt man im allgemeinen ca. 0,5 bis 1 Stunde bei 80 bis 100°C. Anschließend wird unter Erwärmung bei ca. 50 bis 100°C im Vakuum das vorhandene Wasser destillativ abgetrennt. Durch Zudosierung von Ethylenoxid, Propylenoxid oder deren Mischung erfolgt dann die eigentliche Oxalkylierungsreaktion entsprechend den hierbei üblichen Methoden, wobei die Verbindung, die oxalkyliert werden soll, beispielsweise ein C₈-C₁₈-Fettalkohol, bereits vor der Zugabe der Schwefelsäure mit dem Precursor vermischt wird.

Alternativ zu dem zuvor beschriebenen Verfahren kann man auch so vorgehen, daß man auf eine Isolierung des Precursors verzichtet und den Precursor unmittelbar in dem Reaktor für die Oxalkylierung erzeugt. Hierzu wird die Gesamtmenge an zu oxalkylierender Verbindung vorgelegt und mit einer Erdalkaliverbindung oder einer wäßrigen Lösung eines Erdalkalisalzes sowie einer Verbindung der Formel (1) verrührt analog zu dem zuvor beschriebenen Verfahren zur Herstellung des Precursors. Anschließend erfolgt die Bildung des Erdalkalisulfato-Komplexes durch Zugabe der Schwefelsäure. Es wird so eine stabile Öl-in-Wasser-Emulsion erzeugt, wobei die Verbindung der Formel (1) auch als Emulsionsstabilisator dient. Die katalytische Aktivität des Erdalkalisulfato-Komplexes wird durch das Mengenverhältnis Wasser zu Verbindung der Formel (1) bestimmt. Nach der Bildung des Erdalkalisulfato-Komplexes wird das Wasser destillativ entfernt und es erfolgt die Oxalkylierung nach üblichen Verfahren.

Der erfindungsgemäße Precursor bzw. der daraus hergestellte Katalysator in Form des Erdalkalisulfato-Komplexes zeichnet sich durch eine hohe katalytische Aktivität aus. Dies hat zur Folge, daß die für die Oxalkylierung benötigte Zeit deutlich verkürzt wird. Außerdem zeigen die Oxalkylate eine sehr enge Homologenverteilung.

### Beispiel 1

Mengenschema:
0,5 mol Ethercarbonsäure der Formel C₁₂H₂₅O-(C₂H₄O)₃₋₆-CH₂-COOH
3 mol Xylol
0,6 mol C₁₂-Fettalkohol
0,27 mol Calciumhydroxid
1 Gew.-% Celite® J4 (Filterhilfsmittel auf Silikatbasis)

In die Mischung aus Ethercarbonsäure, Xylol und Fettalkohol wird das Calciumhydroxid eingerührt. Nach einer halben Stunde Rührzeit wird bei 100 bis 120°C das Wasser und danach bei 160°C das restliche Xylol abdestilliert. Es werden 1 Gew.-%, bezogen auf den Gesamtansatz, Celite J4 zugegeben und bei 90°C über ein Papierfilter klar filtriert.

24 g des so erhaltenen Precursors mit einem Calciumgehalt von 2,88 % wird mit 630 g C₁₂-Fettalkohol vermischt. Dann werden 3,2 ml 1 molare Schwefelsäure zugegeben und eine halbe Stunde bei 80°C nachgerührt. Unter Vakuum wird dann bei 100°C das Wasser abgezogen. Die Oxethylierung erfolgt dann bei 150 bis 160°C durch Zugabe der gewünschten Menge Ethylenoxid (2; 5 oder 10 mol Ethylenoxid).

### Beispiel 2

Mengenschema:
0,5 mol oxethylierter Fettalkohol der Formel C₁₂H₂₅O-(C₂H₄O)₅H
3 mol Xylol
0,6 mol Fettalkohol der Formel C₁₂H₂₅OH
0,27 mol Calciumhydroxid
1 Gew.-% Celite J4

Oxethylierter Fettalkohol, Xylol und Fettalkohol werden verrührt und das Calciumhydroxid wird dann unter Rühren beigegeben. Nach einer Rührzeit von 30 Minuten bei Raumtemperatur wird im Vakuum bei 100 bis 120°C das Wasser destillativ entfernt. Restmengen an Xylol werden unter Vakuum bei 160°C abdestilliert. Es werden 1 Gew.-% Celite J4 zugegeben und bei 90°C über ein Papierfilter klar filtriert.

### Beispiel 3

Mengenschema:
0,5 mol oxethylierter Fettalkohol der Formel C₁₂H₂₅O-(C₂H₄O)₅H
0,5 mol Fettalkohol der Formel C₁₂H₂₅OH
1,25 mol Calciumoxyd
1 Gew.-% Wasser
1 Gew.-% Celite J4

In die Mischung aus oxethyliertem Fettalkohol und Fettalkohol wird das Calciumoxid und danach 1 Gew.-% Wasser, bezogen auf den Gesamtansatz, eingerührt. Nach einer Rührzeit von 2 Stunden bei 100 bis 120°C wird 1 Gew.-%, bezogen auf den Gesamtansatz, Celite J4 zugegeben und bei 90°C über ein Papierfilter klar filtriert.

### Beispiel 4

In 600 g eines C₁₂-C₁₆-Fettalkohols wird 10,6 g einer wäßrigen Lösung von Calciumacetat (10 g Wasser und 0,6 g Calciumacetat) eingerührt. Zu diesem Ansatz werden 1,8 g eines mit 2 mol Ethlyenoxid oxethylierten C₁₂-C₁₆-Fettalkohol gegeben und die entstehende Emulsion wird 2 Stunden bei 50 bis 70°C gerührt. Dann wird bei 100°C im Vakuum getrocknet, 2 ml einer 1 molaren Schwefelsäure zugegeben, 1 Stunde gerührt und zuletzt das Wasser abdestilliert.

Die Oxethylierung im Anschluß an die in den Beispielen 2 bis 4 beschriebene Herstellung von Precursor und Katalysator erfolgt wie in Beispiel 1 beschrieben.

## Patentansprüche

1. Prcursor für Alkoxylierungskatalysatoren, hergestellt durch Umsetzung eines chelatisierenden anionischen oder nicht-ionischen Phasentransferkatalysators mit einer Erdalkaliverbindung in Gegenwart einer Verbindung, die oxalkyliert werden kann.

2. Precursor nach Anspruch 1, hergestellt durch Umsetzung einer Verbindung der Formel
C₈-C₂₂-Alkyl-O(AO)ₓ-B
als chelatisierender anionischer oder nicht-ionischer Phasentransferkatalysator worin
A -C₂H₄- oder -C₃H₇-,
B Wasserstoff oder eine Gruppe der Formeln -CH₂COOH, -SO₃H oder - PO(OH)₂ und
X eine Zahl von 2 bis 10 bedeutet.

3. Precursor nach Anspruch 1 oder 2, dadurch gekennzeichnet, daß man die Calciumverbindung in Form einer wäßrigen Lösung einsetzt.

4. Alkoxylierungskatalysator, hergestellt durch Zugabe von Schwefelsäure zu einem Precursor nach Anspruch 1 bis 3.

5. Verwendung von Alkoxylierungskatalysatoren, hergestellt durch Zugabe von Schwefelsäure zu einem Precursor nach Anspruch 1 bis 3 bei der Addition von Alkylenoxid an Verbindungen, die sich oxalkylieren lassen.

6. Verwendung von Alkoxylierungskatalysatoren, hergestellt durch Zugabe von Schwefelsäure zu einem Precursor nach Anspruch 1 bis 3 bei der Addition von Alkylenoxid an Fettalkohol.
